# EUROPEAN PATENT APPLICATION

(11) **EP 1 433 841 A1**
(43) Date of publication of application: **30.06.2004**
(21) Application number: 03257897.3
(22) Date of filing: 16.12.2003
(51) Int. Cl.: C12N 1/20, C12N 1/21, C12N 1/36, C12P 21/02

(54) **Method for low-temperature culture of microorganism**

(30) Priority: 17.12.2002 JP 2002365676
(71) Applicant: Nippon Shokubai Co., Ltd., Osaka-shi, Osaka 541-0043 (JP); Imanaka, Tadayuki, Suita-shi, Osaka 565-0873 (JP)
(72) Inventor: Semba, Hisashi, Tsuchiura-shi Ibaraki 300-0810 (JP); Ichige, Eita, Kisaradzu-shi Chiba 292-0806 (JP)
(74) Representative: Bassil, Nicholas Charles

(57) **Abstract**

A method for high-density culture of a microorganism is proposed. This method is characterized by adjusting the cultivation temperature to a level lower than the optimum temperature for growth thereby causing the specific growth rate of the microorganism to reach a level of not more than 20% of the specific growth rate at the optimum temperature for growth. By this method of culture, it is made possible to culture the microorganism at a very high density. Further, this method of culture enables a useful substance, particularly an active type recombinant protein, to be produced in a large amount at a very low cost with high efficiency.

## Description

### BACKGROUND OF THE INVENTION

### Field of the Invention:

This invention relates to a method for culturing a microorganism at a low temperature. This invention further relates to a method for the production of a useful substance by the use of this method of culture.

### Description of the Related Art:

The large-scale cultivation of a microorganism is very useful commercially. The industries utilizing microorganisms are broadly divided into (1) utilization of the cells, (2) utilization of the metabolic products, (3) utilization of the metabolic activity, and (4) genetic engineering. They invariably abound with concrete examples. As concrete examples, (1) dry yeast, lactobacillary preparations, and single cell proteins may be cited concerning the utilization of the cells, (2) alcohol fermentation, lactic acid fermentation, citric acid fermentation, and fermentation of other organic acids, production of vitamins, and production of polysaccharides, enzymes, enzyme inhibitors, and various antibiotic substances concerning the utilization of the metabolic products, and (3) fermentation of amino acids such as glutamic acid and lysine, and conversion of steroids by the microorganism concerning the utilization of the metabolic activity. The utilization of (4) genetic engineering has been attaining remarkable development in recent years and has been finding applications in numerous fields. By the mass culture using the technique of gene recombination, such useful proteins as interferon, erythropoietin, and monoclonal antibody have been being heretofore produced on a commercial scale. Further, attempts to produce novel useful proteins originating in microorganisms, animals, and plants by utilizing the technique of gene recombination have been being pursued extensively.

Heretofore, in culturing a microorganism on a commercial scale, it has been customary to add a source of carbon such as glucose, a source of nitrogen such as peptone, inorganic substances such as SO₄²⁻, PO₄³⁻, and Na⁺, trace elements such as Ca and Zn, and growth factors such as purine and pyrimidine to the culture medium as sources of nutrients. This practice has been at a disadvantage in suffering these sources of nutrients to be consumed and the further growth of the microorganism to be brought to early termination as the logarithmic growth of the microorganism suddenly advances.

For the purpose of solving this problem, the fed-batch culture which consists in continuously or intermittently feeding pertinent sources of nutrients to the culture solution in accordance with the elapse of the cultivation time during the process of the culture of the microorganism has been resorted to. This feeding culture has been finding widespread acceptance because it permits the concentration of a specific component in the culture medium to be controlled arbitrarily, enables the saccharic concentration, for example, to be retained within a fixed range proper for the microorganism being cultured, and consequently affords efficient culture of the microorganism aimed at. This feeding culture, however, has entailed the problem of complicating the apparatus to be used therefor because the control of the timing for feeding a nutrient solution necessitates such operations as detecting the glucose concentration with a glucose sensor and feeding the nutrient solution in compliance with the result of the detection.

In the culture of a microorganism, the adjustment of the dissolved oxygen concentration in the culture solution constitutes an important factor. This is because the oxygen is an extremely important component for a creature which produces energy by aerobic respiration. In the relevant technical field, the optimization of the dissolved oxygen concentration in the culture solution is a very important extrinsic factor. Specifically, when an aerobic microorganism or a facultative anaerobe is to be cultured, this culture is deficient in the efficiency of energy because the oxygen in the culture medium is consumed in accordance as the growth of the microorganism advances and, even if oxygen is supplied by agitation or ventilation for the sake of replenishment, the dissolved oxygen (DO) is wholly depleted early by the growth which surpasses this supply and the microorganism thereafter is made extinct or compelled to produce energy by anaerobic fermentation. In the conventional method for the culture of a microorganism, however, since the microorganism is generally propagated at the maximum specific growth rate, the dissolved oxygen in the culture solution is wholly depleted quickly and, consequently, the degradation of the efficiency of energy is posed as a problem.

In the mass culture of a microorganism, when the growth of the microorganism being cultured is affected by the dissolved oxygen concentration, the technique of precluding the depletion of the dissolved oxygen in the culture medium by furthering the ventilation or by a method of increasing the rotational frequency of the agitation of the culture solution has been known as a solution for the problemmentioned above. When the dissolved oxygen concentration is heightened by such a technique as adding to the amount of ventilation, heightening the oxygen concentration in the air used in the ventilation, or increasing the speed of agitation, since the rate of the growth of the cells being cultured is consequently heightened and the amount of the oxygen consumed is further increased, it becomes difficult to maintain the dissolved oxygen concentration. If the speed of agitation is increased excessively, this excess will be at a disadvantage in inflicting damage upon the cells being cultured. If the amount of ventilation is increased, this increase will be at a disadvantage in proportionately increasing the working volume and disrupting efforts directed toward saving the cost and miniaturizing the apparatus and, moreover, suffering the effervescence of the culture broth to scatter the culture broth and leak it out of the system.

In the present state of affairs, the desirability of developing a method which is capable of culturing a microorganisminexpensively,efficiently,andin high density finds approval. Further, the desirability of developing a method for producing a useful substance efficiently in a large amount by using this method of culturing also finds recognition.

### SUMMARY OF THE INVENTION

The present inventors have tried to culture a microorganism by adjusting the cultivation temperature to a lower level than the optimum temperature for growth so as to lower the specific growth rate of the microorganism to a level of not more than 20% of the specific growth rate at the optimum temperature for growth and consequently succeeded in maintaining an aerobic environment throughout the entire course of the culture and accomplishing a very high final cell density relative to the added sources of nutrients. They have perfected this invention. They have further found that a useful substance produced by a microorganism can be produced inexpensively in a large amount by using the method of culture mentioned above and that, particularly in the case of producing a recombinant protein, the method of culture is effective as well in repressing the formation of an inclusion body. This discovery has resulted in perfecting the present invention.

In accordance with this invention, by a very simple and easy treatment of culturing a microorganism at a low temperature, it is made possible to maintain an aerobic environment and effect the culture with small sources of nutrients at a high density. Further, the method of culture contemplated by this invention proves very useful commercially because it enables a useful substance formed by a microorganism to be produced inexpensively in a large amount. The method of this invention manifests an effect of repressing the formation of an inclusion body particularly when it is adopted for producing a recombinant protein and, therefore, can be expected to find application to a very wide range of fields commercially.

### BRIEF DESCRIPTION OF THE DRAWINGS

Fig. 1 is a diagram showing the relation between the cultivation temperature, and the specific growth rate and the magnitude of specific activity in a flask culture obtained in consequence of the determination of the specific growth rate and the magnitude of specific activity in Example 1.
Fig. 2 is a diagram depicting the analysis of the expression of protein by the use of the SDS-PAGE method with respect to the soluble supernatant and the insoluble precipitate obtained in Example 1.
Fig. 3 is a diagram showing the relation between the cultivation temperature, and the cell density and the dissolved oxygen concentration in a jar fermenter culture, obtained in consequence of the determination of the cell density and the dissolved oxygen concentration in Example 2.

### DETAILED DESCRIPTION OF THE INVENTION

The first aspect of this invention is directed toward a method for the culture of a microorganism, by adjusting the cultivation temperature to a lower level than the optimum temperature for growth so as to lower the specific growth rate of the microorganism to a level of not more than 20% of the specific growth rate at the optimum temperature for growth.

When a microorganism such as a bacterium is grown under new environmental conditions or under new cultivation conditions, generally the microorganism begins propagating after a fixed period called the induction phase, passes the logarithmic phase in which the number of the cells increases, reaches the stationary phase in which the number of live bacteria reaches a fixed level, and eventually arrives at the extinction phase in which the number of cells decreases. Now, the description will be continued by citing a bacterium as an example. The induction phase refers to the period from the time the bacterium is inoculated to the culture medium to the time it is adapted to the new environment. During this period, enzymes and intermediate metabolites are formed and accumulated to an extent of enabling the bacterium to begin growing. During the logarithmic phase, new cellular substances are synthesized at a fixed speed and the number of bacteria increases exponentially. Thereafter, as the depletion of sources of nutrients and the accumulation of harmful substances advance, the fission and the growth of bacteria are moderated and the bacteria which become extinct are increased gradually. In consequence of the equilibration between the bacteria in growth and those in extinction, the number of live bacteria is maintained at a fixed level and the growth of bacteria appears to stop. After the elapse of a fixed duration of the stationary phase, the speed of extinction of cells begins to surpass that of growth thereof and the number of cells begins to decrease, though depending on the kind of bacterium and the conditions of the culture.

For the growth of a microorganism, such physical factors as the cultivation temperature, the dissolved oxygen concentration of the culture solution, and the pH status must be adjusted on the individual basis in addition to the requirement that such sources of nutrients as are needed by the microorganism to be cultured must be included (chemical factor).

The cultivation temperature generally brings a large influence to bear on the growth of a microorganism. From the general point of view, the lower limit of the temperature for the growth is 0°C, i.e. the freezing temperature for the water in the cells or a slightly lower temperature than that and the upper limit of the temperature is fixed by the temperatures for denaturation of such polymer compounds as proteins and nucleic acids. The temperature which allows a given strain to propagate falls in a relatively narrow range. The temperature for the growth of Escherichia coli, for example, has its lower limit in the range of 0 - 15°C, its upper limit at 46°C, and an optimum level in the approximate range of 37 - 42°C. When the microorganisms are classified by the optimum temperature, they fall into cryophilic microorganisms which have optimum temperatures of not higher than 20°C, mesophilic microorganisms which have optimum temperatures in the range of 20 - 45°C, and thermophilic microorganisms which have optimum temperatures of not lower than 45°C. The term "optimum temperature" as used herein refers to the temperature at which a microorganism to be cultured acquires the maximum specific growth rate and the term "specific growth rate" refers to the rate of growth per unit amount of a given microorganism which assumes a numerical value inherent in a given proper and variable with cultivation conditions. The specific growth rate can be determined by the method described in literature (such as "Biophysical Chemistry II - Basis and Exercise - (6^{th} edition) , " Kyoritsu Zensho, pp. 356 - 358). The term "specific growth rate" as used in this invention refers to the magnitude thereof which is obtained in the logarithmic growth phase. Generally, in the culture of a microorganism, the practice of adjusting the cultivation temperature in the neighborhood of the optimum temperature in order that the microorganismmay attain the maximum specific growth rate is a common recourse.

In accordance with this invention, by adjusting the cultivation temperature to a level lower than the optimum temperature for growth and consequently enabling the specific growth rate of a microorganism being cultured to reach a level of not more than 20%, preferably not more than 15%, and particularly preferably not more than 10%, of the specific growth rate at the optimum temperature for growth, it is made possible to repress the metabolism of the microorganism, allay the growth of the microorganism, consequently enable the balance between the consumption of sources of nutrients and the consumption of oxygen to be retained for a very long time, and prevent the degradation of the efficiency of energy. As a result, the microorganism can be cultured for a long time only with the sources of nutrients supplied at the start of the culture and the necessity for replenishing the sources of nutrients during the course of the culture can be obviated. Thus, notwithstanding the specific growth rate is far smaller than the maximum specific growth rate at the optimum temperature for the microorganism, the microorganism can be eventually cultured at a high density at a very low cost.

The "optimum temperature for growth" as used in this invention refers to the temperature at which a microorganism is enabled to acquire the maximum specific growth rate when the conditions including the pH status and the dissolved oxygen concentration and excluding the cultivation temperature are fixed at the time of starting the culture. This invention contemplates adjusting the cultivation temperature to a level lower than the optimum temperature for growth thereby setting the specific growth rate of the microorganism at a level of not more than 20% of the specific growth rate at the optimum temperature for growth because of the possibility that the effect of repressing the metabolism of the microorganism and the consequent effect of improving the balance between the consumption of sources of nutrients and the consumption of oxygen will not attained fully satisfactorily if the specific growth rate exceeds 20% of the specific growth rate at the optimum temperature for growth. Incidentally, for the adjustment of the temperature of the culture solution, any of the popular methods may be adopted.

The microorganisms can be classified by the question whether or not they need oxygen in producing energy. In the case of the bacteria, those which always need oxygen are designated as "aerobic microorganisms" and those which fail to grow in the presence of oxygen and rely on anaerobic fermentation to produce energy are designated as "obligatory anaerobes." The microorganisms which continue respiration in the presence of oxygen and undergo fermentation to produce energy in the absence of oxygen are designated as "facultative anaerobes." The production of energy by respiration is very efficient and advantageous from the viewpoint of energy efficiency as well because the energy produced thereby from fixed amounts of sources of nutrients is ten and odd times the amount obtainable in the case of anaerobic fermentation. It has been known that when a microorganism is anaerobically fermented, it possibly by-produces such organic acids as, for example, acetic acid and consequently inhibits the growth of the microorganism, though depending on the kind of the microorganism. In this invention, the aerobic environment can be maintained for a long time because the good balance between the consumption of oxygen and the consumption of sources of nutrients prevents the dissolved oxygen from being suddenly decreased. In this respect, the microorganism to be used in this invention is preferred to be an aerobic microorganism or a facultative anaerobe. When the execution of this invention fails to maintain the dissolved oxygen concentration at a level exceeding 0 ppm, it is preferred to have the dissolved oxygen concentration adjusted by some other means to a level exceeding 0 ppm. As concrete examples of such other means which is used herein, a method of increasing the amount of ventilation, a method of heightening the speed of agitation, and a method of exalting the air pressure in the jar fermentermaybe cited. Further, for the determination of the dissolved oxygen concentration, any of the popular methods may be adopted without any particular restriction. The dissolved xygen concentration, for example, can be determined with a polarometric type or an electric current type dissolved oxygen meter or a fluorescent type oxygen meter. This invention is characterized by the ability to maintain the aerobic environment for a long time owning to the low-temperature culture and enable the temperature adjustment to be effected more easily then the adjustment of the pH status, the dissolved oxygen, and other cultivation conditions. As a result, this invention can culture a microorganism very simply and easily at a low cost and in a high density.

The microorganism to be subj ected to the growth according to this invention does not need to be particularly restricted but is only required to be effectively cultured by any of the popular methods.

As described above, this invention preferably uses an aerobic microorganism or a facultative anaerobe. It uses Escherichia coli particularly preferably. This is because the Escherichia coli permits large-scale cultivation to be performed easily and enables a gene or a protein aimed at to be produced in a large amount and the method for purifying the produced gene or protein has been established fully satisfactorily. More preferably, recombinant Escherichia coli is used as the microorganism. This is because the use of the recombinant Escherichia coli enables an foreign protein originating in a heterogenic organism to be produced in a large amount.

As regards the mode of the culture contemplated by this invention, the cultivation conditions may be selected in due consideration of the nutritive physiological properties of the microorganism being cultured. In most cases, the culture is carried out in the form of submerged culture. Commercially, the aerated and stirred culture using a jar fermenter proves particularly advantageous.

As the sources of nutrients for the culture solution, a wide variety of sources of nutrients which are generally used for the culture of microorganisms are available. The source of carbon is only required to be assimilable. As concrete examples of the source of carbon, glucose, sucrose, lactose, maltose, molasses, pyruvic acid, and glycerol may be cited. Then, the source of nitrogen is only required to be a nitrogen compound which is assimilable. As concrete examples of the source of nitrogen, such organic nitrogen sources as peptone, meat extract, yeast extract, casein hydrolyzate, and alkali extracts of soybean curd and such inorganic nitrogen sources as ammonia, nitric acid, and salts thereof may be cited. Besides, phosphates, carbonates, sulfonates, salts of magnesium, calcium, potassium, iron, manganese, and zinc, specific amino acids, specific vitamins, and a defoaming agent may be used as occasion demands.

The cultivation time is variable with the kind of a microorganism to be cultured and the cultivation conditions. The culture may be terminated at a proper time which may be selected by waiting the time at which the growth of the microorganism reaches the stationary phase. Further, the pH of the culture medium may be properly changed within the range in which the microorganism is effectively grown. This invention, during the course of the culture, requires the specific growth rate of the microorganism to be adjusted to a level lower than the optimum temperature for growth so that the specific growth rate of the microorganism may reach a level of not more than 20% of the specific growth rate at the optimum temperature for growth. It does not need to restrict the changes in such other conditions as the replenishment of the sources of nutritives and the adjustment of the pH status during the course of the culture than the temperature.

The second aspect of this invention is directed toward a method for the production of a useful substance, by producing the useful substance by culturing a microorganism while adjusting the cultivation temperature to a level lower than the optimum temperature for growth in order that the specific growth rate of the microorganism may be allowed to reach a level of not more than 20% of the specific growth rate at the optimum temperature for the growth.

Apparently, the method for culturing a microorganism at a temperature lower than the optimum temperature is an act of impeding the growth and the metabolism of the microorganism and is liable to be thought improper in the production of a useful substance by the use of a microorganism. Incidentally, in the method of this invention, it is the growth of a microorganism that is repressed at a low temperature. Thus, the yield of cells of the microorganism and the yield of the useful substance are increased on the contrary.

This invention, by culturing a microorganism at a low temperature, is enabled to culture the microorganism at a high density and consequently increase the number of cells of the microorganism to be obtained finally. In consequence of this increase, this invention is naturally expected to bring an additional effect of enabling such useful substances as metabolic products of the microorganism to be produced in a large amount.

Appallingly, when the method of this invention for the culture of a microorganism is adopted in the production of recombinant protein by gene recombination, it is capable of manifesting a synergistic effect and markedly exalting the amount of the protein to be produced. When a foreign protein is mass-produced in recipient such as Escherichia coli, this production more often than not forms the so-called inclusion body, i.e. an inactive cluster of foreign protein because the folding of the product of translation by a molecular chaperon fails to function normally. The inclusion body has resulted from the change of the three-dimensional structure of the product aimed at and the product is prevented from acquiring the activity inherent therein. The product, therefore, creates a need for a refolding treatment. By relying on the method of culture of this invention to produce a recombinant protein, however, it is made possible to repress the formation of an inclusion body and produce the recombinant protein of an active type in a large amount.

As demonstrated in a working example cited herein below, by altering an S-hydroxynitrile lyase gene originating in cassava and indicated by the sequence number 1 (referred to as <400> 1 in the SEQUENCE LISTING described below and hereinafter occasionally referred to as "Ori gene"), transforming Escherichia coli with an altered gene indicated by the sequence number 2 (referred to as <400> 2 in the SEQUENCE LISTING described below and hereinafter occasionally referred to as "Semba gene"), and culturing the transformed Escherichia coli by the method of culture contemplated by this invention, it is made possible to accomplish a high cell density, repress the formation of an inclusion body, and produce an active type S-hydroxynitrile lyase in a large amount.

In the light of the point described above, a more preferred mode of this invention consists in a method for producing a useful protein by using a recombinant microorganism and a particularly preferred mode thereof resides in a method of using Escherichia coli as the microorganism. Here, the mode of the gene recombination does not need to be particularly restricted but may be properly decided by the nature of a microorganism to be used as a recipient. Further, the protein to be produced does not need to be particularly restricted but may be arbitrarily selected from among the proteins which originate in microorganisms, plants, and animals.

The useful substance contemplated by this invention does not need to be particularly restricted but may be in any form so long as it is a substance that can be produced by utilizing a microorganism. For one example of the useful substance, a microorganism cell itself may be cited. A dry yeast, for example, maybeproduced. Further, since themethodof culture of this invention is capable of exalting the final cell density of a microorganism, it enables even a substance which is generally produced by the metabolism of a microorganism to be produced with very high efficiency. As concrete examples of the useful substance of this sort, vitamins, enzymes, antibiotic substances, amino acids, fatty acids, and alcohols may be cited. The production of a useful substance can be attained by utilizing the biotic activity possessed by a microorganism obtained by the method of this invention. The product of the conversion of steroid by the microorganism, for example, can be produced.

### Examples

Now, this invention will be described with reference to working examples below. This invention is not limited to the following examples.

### [Example 1]

For the purpose of inserting a Semba gene indicated by the sequence number 2 into a recombinant vector, a PCR primer (Sense: GGG CAT ATG GTT ACT GCA CAC TTC GTT CTG ATT CAC ACC (sequence number 3) ; Antisense: CCC GGA TCC TTA AGC GTA TGC ATC AGC AAC TTC TTG CAG AAT (sequence number 4) ) was synthesized and a PCR reaction was performed with the Semba gene as a template. The gene used herein as the template was prepared by dividing the Semba gene into several oligomers, entrusting Espec Origo Service K.K. to form genes respectively from the divided oligomers, and connecting these origomers by the PCR method. The primers mentioned above were made to include the restriction sites of the restriction enzymes NdeI and BamHI. By using the restriction enzymes NdeI and BamHI, it was made possible to connect the Semba gene and the recombinant vector.

The PCR reaction used in this case consisted of one cycle of incubation at 98°C for five minutes, 30 cycles of incubation (each using 98°C for 30 seconds, 57°C for 15 seconds, and 74°C for 30 seconds), and a final cycle of incubation using 4°C for five minutes to one hour.

When the product of the PCR reaction was assayed by a popular method of electrophoresis, it was confirmed to contain a gene corresponding to the Semba gene.

For the purpose of inducing the expression of the Semba gene mentioned above in Escherichia coli, the pET21a vector possessing a T7 promoter having a potent gene expressing ability (made by Novagen Corp.) was subjected to subcloning.

The method adopted for the subcloning comprised treating the pET21a vector and the Semba gene with restriction enzymes NdeI and BamHI and subjecting them to ligation by a specified method using a Ligation high Kit (made by Toyobo K.K.).

The Escherichia coli DH5α competent cell was transformed by using a pET21a mixed solution which had a Semba gene incorporated therein and had been obtained as described above. Thereafter, the product of this transformation was spread on an LB agar culture medium and incubated thereon at 37°C for 12 - 16 hours.

From the colonies of the transformed Escherichia coli grown on the LB agar culture mediummentioned above, 10 colonies were selected arbitrarily and subjected to identification of colony by the colony PCR method.

The colonies which had been confirmed to have admitted the insert were individually cultured in an LB liquid culture medium at 37°C for eight hours. From each of the formed culture, a plasmid was recovered in the same manner as described above. The plasmids were designated as pET21a/Semba.

Escherichia coli strain BL21 (DE3) (made by Novagen Corp.) was transformed by using pET21a/Semba thus obtained. The gene recombinant Escherichia coli strain thus obtained was designated as BL21/pET21a/Semba.

The recombinant Escherichia coli strain (BL21/pET21a/Semba) organized as described above was cultured by the following method to obtain a recombinant protein.

First, the portion of the strain equivalent to one colony was inoculated to 5 mL of an LB culture medium in a 15-mL test tube and precultured at a cultivation temperature of 37°C, at a shaking rate of 170 rpm, for 12 hours. Subsequently, in a 500-mL Sakaguchi flask, each precluture solution 2 mL in volume was inoculated to 100 mL of a nutritive culture medium (composition of culture medium: 40 g of glycerol, 10 g of ammonium sulfate ((NH₄)₂SO₄), 2 g of potassium dihydrogen phosphate (KH₂PO₄), 6 g of dipotassium hydrogen phosphate (K₂HPO₄), 40 g of yeast extract, 1 g of magnesium sulfate (MgSO₄), 20 drops of Adekanol in 1 L of distilled water, pH 6), isopropyl-β-D-thiogalactopyranoside (IPTG), a protein expression inducing agent, was added thereto till the final concentration thereof reached 1 mM, and they were subjected to production culture at a shaking rate of 130 rpm at a cultivation temperature of 27°C, 22°C, 19°C, and 17°C till the culture reached the stationary state. Generally, the growth of a bacterium possibly occurs in a non-logarithmic form after it has proceeded in a logarithmic form. In this invention, the time at which the non-logarithmic growth ceases to proceed is regarded as the point of reaching the stationary phase.

After the culture mentioned above, the final cell concentration of the produced culture broth was measured by determining the absorbance of the solution at 660 nm. From the numerical value obtained as described above, the specific growth rate during the logarithmic phase of growth was calculated by a popular method. The cell pellet was collected by centrifugation of the culture broth and homogenized. Then the homogenate was divided into a soluble supernatant and an insoluble precipitate fraction.

By the method of literature (JP-A-2000-189160) using the soluble supernatant obtained as described above, the activity of S-hydroxynitrile lyase (hereinafter occasionally referred to as "SHNL") was measured.

### [Comparative Example 1]

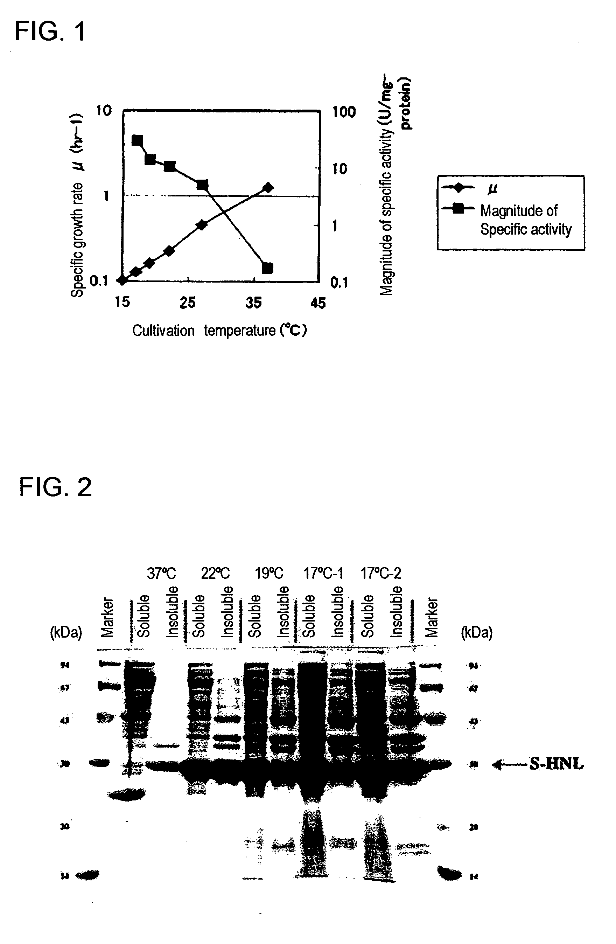

The same gene recombinant Escherichia coli BL21/pET21a/Semba as used in Example 1 was subjected to culture, and determination of final cell density and SHNL activity by following the procedure of Example 1 while changing the cultivation temperature to 37°C. The results are shown in Table 1 and Fig. 1.

**[Table 1]**

| Strain | Final cell density (OD660) | Magnitude of activity (U/mL-broth) | Magnitude of specific activity (U/mg-protein) |
|---|---|---|---|
| BL21/pET21a/Semba 37°C | 10.6 | 230 | 0.17 |
| BL21/pET21a/Semba 27°C | 38.08 | 11140 | 5 |
| BL21/pET21a/Semba 22°C | 27.88 | 34658 | 10.33 |
| BL21/pET21a/Semba 19°C | 37.12 | 79180 | 13.52 |
| BL21/pET21a/Semba 17°C | 51.12 | 215600 | 29.08 |

The soluble supernatant and the insoluble precipitate obtained as described above were assayed for protein by a popular method SDS-polyacrylamide gel electrophoresis (SDS-PAGE). Here, 20 µL per well was applied as diluted so as to fix the OD value in the SDS-PAGE analysis. The results are shown in Fig. 2.

### [Example 2]

The BL21/pET21a/Semba was cultured by the following method with the object of analyzing the effect exerted by the method of low-temperature culture on the changes of the dissolved oxygen concentration in the culture solution.

First, in a 500-mL Sakaguchi flask, the portion of the recombinant Escherichia coli BL21/pET21a/Semba organized in Example 1 which was equivalent to one colony was inoculated to 100 mL of the same nutritive culture medium as used in Example 1 and precultured at a cultivation temperature of 37°C at s shaking rate of 130 rpm for 12 hours. Subsequently, in a 2-L jar fermenter, 25 ml of each of the precultured solution was inoculated to 1200 mL of the nutritive culture medium, isopropyl-β-D-thiogalactopyranoside (IPTG), a protein expression inducing agent was added thereto till the final concentration thereof reached 1 mM, and they were subjected to production culture at s agitation rate of 680 rpm, a rate of aeration of 1 vvm, and a temperature of 17°C. The culture was continued till the growth reached the stationary phase.

During the course of the culture, the culture broth was sampled at intervals of 6 hours. The cell pellet was collected by centrifugation of the culture broth and homogenized. Then the homogenate was divided into a soluble supernatant and an insoluble precipitate fraction.

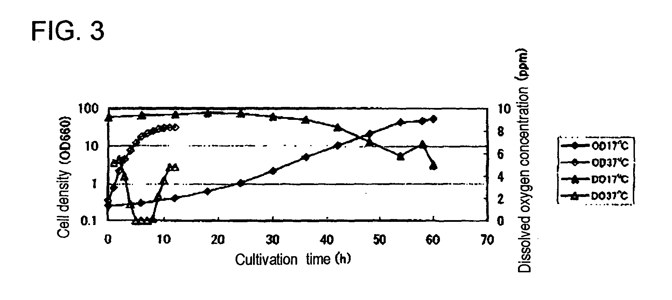

The samples of the culture broth were obtained at intervals of 6 hours during the course of the culture mentioned above and cell density was measured. Dissolved oxygen concentration (DO) was measured by the use of a dissolved oxygen meter provided with an oxygen electrode (made by Marubishi BioEngineering K.K.), and it recorded every 6 hours during the culture. The results are shown in Fig. 3.

### [Comparative Example 2]

The same recombinant Escherichia coli strain BL21/pET21a/Semba as used in Example 2 was cultured, and the final cell density and the dissolved oxygen concentration were measured by following the procedure of Example 2 while changing the cultivation temperature to 37°C. The results are shown in Fig. 3.

### Results:

It is seen from Fig. 1 that the specific growth rate at 17°C was 0.126 (hr⁻¹), i.e. about 10% of that obtained at 37°C, which was 1.271 (hr⁻¹). The final cell density at that temperature, however, had a magnitude about five times that obtained at 37°C as noted from Table 1. Then, the dissolved oxygen concentration in the culture solution, as noted from Fig. 3, reached 0 ppm after the elapse of five hours after the start of the culture at 37°C, whereas it did not reach 0 ppm during the course of the culture at 17°C. These results indicate that since the low-temperature culture maintained a very good balance between the supply and the consumption of oxygen, the efficiency of the consumption of energy by the microorganism did not decrease and the microorganism could be cultured at a high density without particularly requiring the control of the dissolved oxygen concentration which had been a task imposed on the ordinary culture.

It is noted from Table 1 and Fig. 1 that by performing the culture at the low temperature, it was made possible to exalt the magnitude of activity and the magnitude of specific activity greatly in spite of a decrease in the specific growth rate. The specific activity exhibited when the culture was performed at 37°C was only 0.17 (U/mg-protein). By setting the cultivation temperature at 17°C, the specific activity was made to increase to 29.08 (U/mg-protein), namely to 170 times the value obtained at 37°C. One more reason which can be adduced for the acquisition of such a high specific activity is that the amount of the active type target protein contained in the soluble protein had increased.

The terms "insoluble" and "soluble" indicated in Fig. 2 refer respectively to the insoluble precipitate and the soluble supernatant which were obtained by centrifugation in Example 1. The SHNL was present in both the insoluble precipitate and the soluble supernatant. Here, the SHNL in the insoluble precipitate is thought to have been an inactive body forming an inclusion body. It is noted from Fig. 2 that the difference in temperature resulted in the variation of the proportions of the soluble and insoluble fractions in the produced SHNL. At 37°C, a band appeared in a substantially 100% insoluble fraction. At 22°C and 19°C, the soluble fraction and the insoluble fraction formed a ratio of nearly half and half. At 17°C, the proportion of the soluble SHNL markedly increased. In the low-temperature culture, the formation of an insoluble inclusion body due to the recombinant protein was repressed and the proportion of the active body could be increased. Further, since the low-temperature culture allowed the final cell density to be largely increased, the soluble recombinant protein, which was the active type, could be produced in a large amount owing to the synergistic effects.

SEQUENCE LISTING

<110> Nippon Shokubai Co., Ltd.

<120> Method for low-temperature culture of a microorganism

<130> F2003-056

<160> 4

<170> PatentIn Ver. 2.1

<210> 1
<211> 777
<212> DNA
<213> Manihot esculenta Crantz

<400> 1
atggtaactg cacattttgt tctgattcat accatttgcc atggtgcatg gatttggcat 60
aagctcaaac cagcccttga gagagctggc cacaaagtca ctgcactgga catggcagcc 120
agcggcattg acccaaggca aattgagcag attaattcat ttgatgaata ctctgaaccc 180
ttattgactt tcttggagaa actccctcaa ggggaaaagg tcatcattgt tggtgagagc 240
tgtgcagggc tgaatattgc tattgctgct gatagatacg ttgacaaaat tgcagctggt 300
gttttccaca attccttatt gccagacacc gttcatagcc catcttacac tgtggaaaag 360
cttttggagt cgtttcctga ctggagagac acagagtatt ttacgttcac taatatcact 420
ggagagacaa ttacaacaat gaagctgggc ttcgtacttc tgagggaaaa tttatttacc 480
aaatgcactg atggggaata tgaactggca aaaatggtaa tgaggaaggg atcactgttt 540
caaaatgttt tggctcagag accgaagttc accgaaaaag gttacggatc aattaagaaa 600
gtttatattt ggaccgatca agacaaaata tttttaccag actttcaacg ctggcaaatt 660
gcaaactaca aaccagacaa ggtttatcag gttcaaggtg gagatcataa gctccagctt 720
acaaaaactg aggaggtagc tcatattctc caagaggtgg ctgatgcata tgcttga 777

<210> 2

<211> 777
<212> DNA
<213> Artificial Sequence

<220>
<223> Description of Artificial Sequence:CDS

<400> 2
atggttactg cacacttcgt tctgattcac accatttgtc acggcgcatg gatttggcac 60
aaactgaaac cggccctgga acgtgctggc cacaaagtta ctgcactgga catggcagcc 120
agtggcattg acccgcgtca aattgaacag atcaactctt tcgatgaata ctctgaaccg 180
ctgctgactt tcctggaaaa actgccgcaa ggcgaaaagg ttatcattgt tggtgaaagc 240
tgtgcaggcc tgaacattgc tattgctgct gatcgttacg ttgacaaaat tgcagctggc 300
gttttccaca actccctgct gccggacacc gttcacagcc cgtcttacac tgttgaaaag 360
ctgctggaat cgttcccgga ctggcgtgac acagaatatt tcacgttcac caacatcact 420
ggcgaaacca tcactaccat gaaactgggt ttcgttctgc tgcgtgaaaa cctgttcacc 480
aaatgcactg atggcgaata tgaactggca aaaatggtta tgcgcaaggg ctctctgttc 540
caaaacgttc tggctcagcg tccgaagttc actgaaaaag gctacggctc tatcaagaaa 600
gtttatattt ggaccgatca agacaaaata ttcctgccgg acttccaacg ctggcaaatt 660
gcaaactaca aaccggacaa ggtttatcag gttcaaggcg gcgatcacaa gctgcagctg 720
acaaaaactg aagaagtagc tcacattctg caagaagttg ctgatgcata cgcttaa 777

<210> 3
<211> 39
<212> DNA
<213> Artificial Sequence

<220>
<223> Description of Artificial Sequence:Primer

<400> 3

gggcatatgg ttactgcaca cttcgttctg attcacacc 39

<210> 4
<211> 42
<212> DNA
<213> Artificial Sequence

<220>
<223> Description of Artificial Sequence:Primer

<400> 4
cccggatcct taagcgtatg catcagcaac ttcttgcaga at 42

The entire disclosure of Japanese Patent Application No. 2002-365676 filed on December 17, 2002 including specification, claims, drawings, and summary are incorporated herein by reference in its entirety.

## Claims

1. A method for the culture of a microorganism, by adjusting the cultivation temperature to a level lower than the optimum temperature for growth thereby causing the specific growth rate of said microorganism to reach a level of not more than 20% of the specific growth rate at the optimum temperature for growth.

2. A method according to claim 1, wherein the dissolved oxygen concentration in the culture solution is adjusted to a level exceeding 0 ppm.

3. A method according to claim 1 or 2, wherein said microorganism is Escherichia coli.

4. A method for the production of a useful substance, by effecting the production of the useful substance by culturing a microorganism with the cultivation temperature adjusted to a level lower than the optimum temperature for growth thereby causing the specific growth rate of said microorganism to reach a level of not more than 20% of the specific growth rate at the optimum temperature for growth.

5. A method according to claim 4, wherein said microorganism is a gene recombinant microorganism.

6. A method according to claim 4 or 5, wherein said microorganism is Escherichia coli.
